# FASCICULE DE BREVET EUROPEEN

(11) **EP 0 557 489 B1**
(45) Date de publication et mention de la délivrance du brevet: **09.10.1996**
(21) Numéro de dépôt: 92919214.4
(22) Date de dépôt: 27.08.1992
(51) Int. Cl.: A61K 7/00, A61K 9/51

(54) **COMPOSITION POUR UN TRAITEMENT COSMETIQUE ET/OU PHARMACEUTIQUE DE LONGUE DUREE DES COUCHES SUPERIEURES DE L'EPIDERME PAR UNE APPLICATION TOPIQUE SUR LA PEAU**
LANGWIRKENDE TOPISCHE ZUSAMMENSETZUNG FÜR KOSMETISCHE UND/ODER PHARMAZEUTISCHE ANWENDUNG AUF DER ÄUSSEREN SCHICHT DER HAUT
COMPOSITION PROVIDING A LASTING COSMETIC AND/OR PHARMACEUTICAL TREATMENT OF THE UPPER EPIDERMAL LAYERS BY TOPICAL APPLICATION ON THE SKIN

(30) Priorité: 13.09.1991 FR 9111344
(43) Date de publication de la demande: 01.09.1993
(73) Titulaire: L'OREAL, F-75008 Paris (FR)
(72) Inventeur: RIBIER, Alain, F-75014 Paris (FR); RICHART, Pascal, F-94240 L'Hay-les-Roses (FR); HANDJANI, Rose-Marie, F-75014 Paris (FR)
(74) Mandataire: Peuscet, Jacques
(86) Numéro de dépôt international: FR9200824
(87) Numéro de publication internationale: WO9305753

(56) Documents cités:
- EP-A- 0 254 447
- EP-A- 0 274 961
- EP-A- 0 409 690
- GB-A- 2 166 651
- US-A- 3 965 033

## Description

La présente invention concerne une composition pour un traitement cosmétique et/ou pharmaceutique de longue durée des couches supérieures de l'épiderme par une application topique sur la peau.

Il est connu depuis longtemps en cosmétique et/ou en pharmacie, d'appliquer sur la peau des huiles actives par elles-mêmes et/ou contenant un actif. Ces huiles peuvent être utilisées telles quelles ; elles sont le plus souvent sous forme d'émulsion eau dans l'huile ou huile dans l'eau. Elles peuvent également, de façon connue, être appliquées sous forme encapsulées dans des microcapsules de gélatine ou d'autres polymères que l'on écrase sur la peau pour libérer les huiles, ces microcapsules ayant, en général, des dimensions comprises entre 5.10⁴ et 2.10⁶ nanomètres (voir, par exemple, US-A 4 976 961). Mais les huiles appliquées restent en majeure partie à la surface de la peau où elles peuvent être rapidement éliminées par simple frottement, par exemple, au contact des vêtements, ou par lavage.

Par ailleurs, on connaît, sous le nom de nanoparticules, des particules de colloïdes, qui ont des dimensions beaucoup plus faibles que les microparticules, puisque leur taille est, en général, de l'ordre de 10 à 1000 nm ; dans ces nanoparticules, on piège un principe actif par encapsulation et/ou par adsorption et/ou absorbtion (voir J. Keuter, J. microencapsulation 1988 Vol 5, pages 115-127). Le terme de nanoparticules est générique et recouvre, d'une part, les nanosphères et, d'autre part, les nanocapsules ; on désigne par le terme "nanosphères", les nanoparticules constituées par une matrice polymérique poreuse sur laquelle l'actif est absorbé et/ou adsorbé et par le terme "nanocapsules" les nanoparticules constituées par une membrane polymérique, qui entoure un coeur formé par le principe actif. Les polymères utilisables pour la fabrication des nanocapsules peuvent être, de façon connue, soit des matériaux biodégradables, soit des matériaux non biodégradables.

Dans EP-A-0 274 961, est décrite une composition thérapeutique et/ou cosmétique contenant des nanosphères sur lesquelles des actifs sont fixés par adsorption ou réaction chimique.

Dans la demande de brevet français 90 034 418, la demanderesse a décrit une composition cosmétique pour application topique contenant des nanocapsules en polymère biodégradable encapsulant une phase huileuse, qui contient un actif. La demanderesse a trouvé que les nanocapsules pénètrent dans la peau et libèrent leur contenu en majeure partie dans l'épiderme ; les nanocapsules étant en polymère biodégradable, se dégradent rapidement dans l'épiderme, sous l'action des enzymes qui y sont présentes et libèrent la phase huileuse qu'elles contiennent. Dans cette composition, la phase huileuse étant libérée in situ dans l'épiderme, ne peut donc plus être éliminée par simple frottement ou par lavage. L'actif a un effet maximum immédiat, ce qui est avantageux dans de nombreux traitements où une activité immédiate est souhaitée, tels que les traitements antiinflammatoires.

Mais dans un certain nombre de traitements cosmétiques et/ou pharmaceutiques, on souhaite que les actifs aient une action prolongée dans le temps, sans qu'il soit nécessaire de renouveler trop fréquemment l'application. C'est le cas, par exemple, pour la protection de la peau par des filtres solaires, pour la coloration directe de la peau par des colorants ou des pigments et pour le traitment de la peau par des agents déodorants, humectants ou capteurs de radicaux libres.

Selon la présente invention, on a trouvé que l'on pouvait obtenir une composition contenant des nanocapsules, dans laquelle les actifs ont une action prolongée dans le temps, en utilisant des nanocapsules de polymère non-biodégradable.

La présente invention a donc pour premier objet, l'utilisation de nanocapsules de polymère non-biodégradable encapsulant une phase huileuse contenant au moins un actif sous forme d'huile et/ou au moins un actif contenu dans une huile-support non active ou une huile active, l'actif étant choisi parmi ceux ayant une action pharmaceutique se manifestant dans les couches supérieures de l'épiderme, pour l'obtention d'un médicament pour le traitement des couches supérieures de l'épiderme par application topique sur la peau, chaque application devant avoir une action prolongée dans le temps, les nanocapsules ayant des dimensions comprises entre 100 et 1 000 nm.

La présente invention a pour second objet un procédé de traitement cosmétique des couches supérieures de l'épiderme par application topique sur la peau, caractérisé par le fait qu'on applique dans un véhicule approprié sur la peau des nanocapsules de polymères non-biodégradables encapsulant au moins un actif sous forme d'huile et/ou au moins un actif contenu dans une huile-support non active ou une huile active, l'actif étant choisi parmi ceux ayant une action cosmétique sur les couches supérieures de l'épiderme, les nanocapsules ayant des dimensions comprises entre 100 et 1 000 nm.

Au moins un des actifs est, de préférence, choisi parmi ceux devant avoir sur la peau une action cosmétique et/ou pharmaceutique de longue durée. On entend par "action de longue durée" une activité persistant pendant au moins une heure.

On a constaté que les nanocapsules ayant des dimensions comprises entre 100 et 1000 nm sont suffisamment petites pour pouvoir se glisser entre les cornéocytes les plus superficiels du stratum corneum, sans atteindre toutefois l'épiderme vivant. Les nanocapsules pénètrent donc dans les premières àssises mal jointes du stratum corneum et échappent ainsi aux risques d'élimination rapide de la surface de la peau par simple frottement. Elles seront naturellement éliminées au fur et à mesure de la desquamation normale de la peau.

Le poids des nanocapsules chargées d'une phase huileuse utilisées constitue avantageusement de 0,1 à 20% du poids total de la composition et, de préférence de 0,5 à 10% en poids.

Par polymères non-biodégradables, on entend, dans la présente demande de brevet, les polymères formant des membranes de nanocapsules qui ne sont pas dégradées par les enzymes de la peau. Ces membranes, selon le choix du polymère peuvent être semiperméables.

Les membranes des nanocapsules selon l'invention doivent également être très résistantes à leur environnement après formulation de la composition, que cet environnement soit aqueux ou huileux.

Parmi les polymères ayant les caractéristiques ci-dessus, on peut citer :
- les copolymères de chlorure de vinyle et d'acétate de vinyle, par exemple ceux commercialisés sous la dénomination "RHODOPAS AX 8515" par la société RHONE POULENC ;
- les copolymères d'acide méthacrylique et d'ester méthylique d'acide méthacrylique, par exemple ceux commercialisés sous la dénomination "EUDRAGIT L 100" par la société ROHM PHARMA ;
- l'acéto-phtalate de polyvinyle ;
- l'acéto-phtalate de cellulose ;
- le copolymère polyvinylpyrrolidone-acétate de vinyle réticulé ;
- les polyéthylènevinylacétates ;
- les oligomères styrène alkylalcool ;
- les copolymères d'acide et d'ester méthacrylique ;
- les polyacrylonitriles ;
- les polyacrylamides ;
- les polyéthylèneglycols ;
- les poly-(méthacrylate d'hydroxyéthyle) ;
- les dérivés de cellulose ;
- les polyamides ;
- les polyéthylènes ;
- le polypropylène ;
- les organopolysiloxanes dont les polydiméthylsiloxanes;
- la gélatine.

Les nanocapsules obtenues ont, de façon générale, une membrane transparente. Dans ces conditions, on a observé que les colorants directs de la peau et les filtres antisolaires peuvent respectivement jouer leur rôle de coloration ou de protection de la peau sans qu'ils soient libérés des nanocapsules. On a constaté, que, malgré une répartition discontinue du filtre antisolaire ou du colorant, on obtenait un effet continu, c'est-à-dire, une protection ou une coloration uniforme de la peau.

Dans la mesure où la membrane est semiperméable, elle permet des échanges d'eau ou d'humidité avec son environnement. Par conséquent, si les nanocapsules contiennent comme actif un humectant, celui-ci pourra remplir sa fonction et hydrater la peau.

La membrane pouvant être semi-perméable sous l'action du sebum, des agents bactéricides, des déodorants et des agents antiséborrhéiques peuvent être encapsulés dans les nanocapsules. Ils seront libérés au cours du temps sous l'action du sébum.

La phase huileuse encapsulée dans les nanocapsules peut contenir une huile active. Cette huile peut être notamment un filtre antisolaire UVA et/ou UVB, par exemple, à base de p-méthoxycinnamate d'éthylhexyle (vendu sous la dénomination "PARSOL MCX") ou de tertio-butyl-4 méthoxy-4' dibenzoylméthane (vendu sous la dénomination "PARSOL 1789"), et/ou une huile essentielle bactéricide et/ou fongicide, telle que l'huile de thym, une huile à activité humectante telle que le tétraéthyl-2 hexanoate de pentaérythritol et une huile à activité antiradicaux libres telle que l'alpha-tocophérol.

Les huiles-supports non-actives sont, de préférence, choisies parmi les triglycérides simples ou modifiés, en particulier par oxyéthylénation, les huiles de silicone volatiles et les huiles minérales.

L'actif est, de préférence, un actif oléophile qui se dissout dans l'huile. Mais il peut également être sous forme de dispersion, de suspension ou d'émulsion.

Dans la phase huileuse, on peut, selon l'invention, introduire au moins un actif non huileux dans une huile-support et/ou dans une huile active. Cet actif, quelle que soit sa présentation, peut être un agent humectant tel que l'acide hyaluronique, l'acide orotique, ou un lipoprotide, un antiacnéique, un liporégulateur tel qu'un extrait de Centella Asiatica ou le gamma-orizanol, un antivieillissement tel que le palmitate de vitamine A, un colorant, un pigment, un émollient tel qu'un ester d'isopropyle, un kératolytique tel que l'acide rétinoïque ou l'acide n -octanoyl 5 -salicylique.

Selon l'invention, la composition peut également contenir des nanocapsules de polymère(s) biodégradable(s) encapsulant une phase huileuse contenant au moins un actif qui, de préférence, a une action immédiate après application. On peut ainsi obtenir une composition contenant à la fois un (ou des) actif(s) à action immédiate et un (ou des) actif(s) à action prolongée.

Les compositions selon l'invention peuvent se présenter sous forme de sérum, de lotion, de gel aqueux, hydroalcoolique ou huileux, d'émulsion eau dans l'huile ou huile dans l'eau, de dispersions aqueuses de vésicules lipidiques constituées de lipides ioniques ou non-ioniques ou du mélange des deux (vésicules mixtes) ; ces dispersions vésiculaires pouvant en outre comporter éventuellement une huile dispersée selon l'enseignement de FR-A 2 485 921.

La composition peut également contenir des adjuvants cosmétiquement et/ou pharmaceutiquement acceptables, qui forment le véhicule de la composition après formulation. Parmi ces adjuvants, on peut citer les corps gras, les huiles, la vaseline, les conservateurs, les agents épaississants, les colorants et les parfums.

Les nanocapsules selon l'invention sont, de préférence, préparées de façon connue par le procédé par précipitation de polymères préformés de H. Fessi et JP. Devissaguet décrit dans EP-A 0 274 961.

Dans ce cas, les nanocapsules sont obtenues par précipitation du polymère autour d'une dispersion de gouttelettes huileuses en injectant dans une phase aqueuse contenant ou non un (ou plusieurs) agent(s) tensioactif(s), un mélange constitué par l'(les) huile(s) à encapsuler, au moins un polymère et au moins un solvant, puis en évaporant le solvant. Si on le désire, on élimine tout ou partie du solvant de manière à obtenir une suspension colloïdale de concentration déterminée en nanocapsules ou à obtenir, après addition de substances stabilisantes, une poudre de nanocapsules.

D'autres procédés connus de fabrication de nanocapsules peuvent également être utilisés.

Les exemples donnés ci-après, à titre purement illustratif et non limitatif, permettront de mieux comprendre l'invention.

### Exemples 1 à 5 - Préparation de nanocapsules.

Dans un bécher de 100 ml, on dissout 125 mg de condensat d'oxyde d'éthylène et d'oxyde de propylène dans la proportion en poids 20/80, de poids moléculaire moyen 1750 et 8350 respectivement, commercialisé sous la dénomination "PLURONIC F 68" par la société BASF, dans 50 ml d'eau déminéralisée, sous l'agitation donnée par un barreau magnétique tournant à 400t/min.

Dans cette phase aqueuse thermostatée à 40°C, on verse lentement 25 ml d'alcool absolu dans lequel on a préalablement dissous, à la température de 40°C, le polymère non-biodégradable et l'actif (ou les actifs) utilisés dans l'exemple considéré.

On maintient l'agitation pendant 20 minutes à la température de 40°C puis on laisse revenir le mélange à température ambiante.

On transfère alors la dispersion de nanocapsules obtenue dans un ballon rond de 250 ml que l'on place sur un évaporateur rotatif, et on évapore l'alcool et une partie de l'eau jusqu'à obtenir 10 ml d'une dispersion colloïdale blanche, fluide, de nanocapsules. On mesure le diamètre moyen et la polydispersité (IP) au "nanosizer"(Granulomètre Coultronics).

L'indice IP est évalué sur une échelle de 0 à 9, 0 correspondant à une homogénéité quasi parfaite de la taille des particules et 9 indiquant un rapport élevé entre la taille des plus grosses particules et celle des plus petites. Les polymères et les actifs utilisés ainsi que le diamètre moyen et la polydispersité des nanocapsules obtenues sont donnés dans le tableau I ci-après.

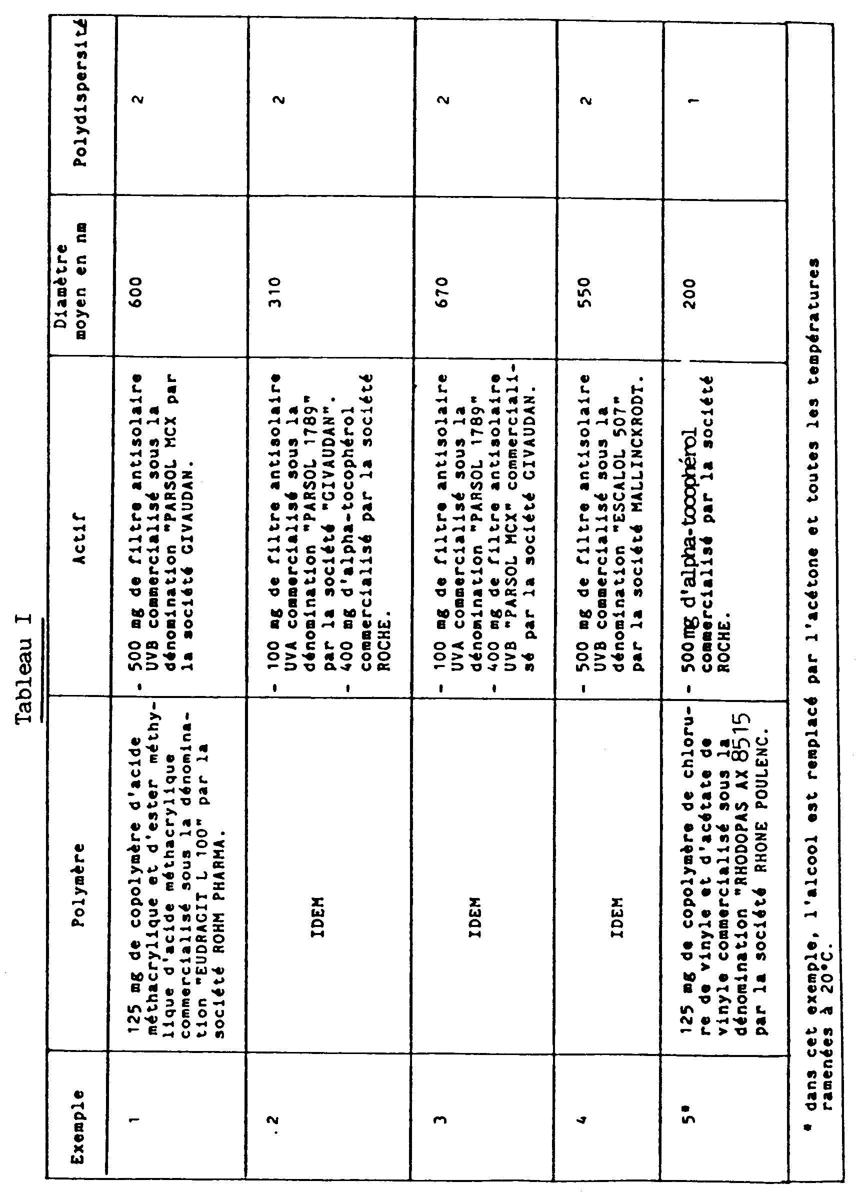

### Exemple 6 (comparatif).

On a comparé l'indice de protection apporté par deux compositions ; une composition A contenant un filtre antisolaire et une composition B selon l'invention, contenant le même filtre antisolaire encapsulé dans des nanocapsules.

### a) Formulations

| Composition A : | |
|---|---|
| - Filtre antisolaire UVB commercialisé sous la dénomination "PARSOL MCX" par la société GIVAUDAN | 4 g |
| - Mélange d'alcools cétylstéarique et cétylstéarique oxyéthyléné à 33 moles d'oxyde d'éthylène (80/20 en poids) | 2,8 g |
| - Mono-et distéarate de glycérol (40/50 en poids) | 0,8 g |
| - Alcool cétylique | 0,6 g |
| - Polydiméthylsiloxane | 0,6 g |
| - Huile de vaseline | 6 g |
| - Parahydroxybenzoate de butyle | 0,08 g |
| - Eau déminéralisée qsq | 100 g |

| Composition B : | |
|---|---|
| - Dispersion de nanocapsules préparées selon l'exemple 1 | 80 g |
| - Mélange d'alcools cétylstéarique et cétylstéarique oxyéthyléné à 33 moles d'oxyde d'éthylène (80/20 en poids) | 2,8 g |
| - Mono-et distéarate de glycérol (40/50 en poids) | 0,8 g |
| - Alcool cétylique | 0,6 g |
| - Polydiméthylsiloxane | 0,6 g |
| - Huile de vaseline | 6 g |
| - Parahydroxybenzoate de butyle | 0,08 g |
| - Eau déminéralisée qsq | 100 g |

### b) Essais

Les deux compositions ont été appliquées de façon régulière sur le dos de dix sujets à raison de 2 mg/cm².

On soumet ensuite la peau à un rayonnement UVB 15 à 20 min après application des compositions.

On place sur la zone à exposer une plaque opaque munie d'ouvertures circulaires de 15 mm de diamètre. Ces ouvertures sont fermées les unes après les autres à intervalles déterminés de façon à avoir des zones de peau ayant reçu des doses de rayonnement différentes.

Les observations sont effectuées (24 ± 2) heures après l'exposition au rayonnement. On détermine le premier cercle juste visible, mais nettement délimité et on détermine la dose d'énergie en UV-B reçue (MED).

L'indice de protection est le rapport de la MED en présence du produit testé et de la MED en l'absence de produit antisolaire.

Les résultats obtenus avec les compositions testées sont les suivants :

| Composition | Indice de Protection |
|---|---|
| A | 3,85 |
| B | 5,30 |

Ces résultats établissent que la composition selon l'invention a conduit à une augmentation significative de l'indice de protection pour une même concentration de filtre antisolaire.

## Revendications

1. Utilisation de nanocapsules de polymère non-biodégradable encapsulant une phase huileuse contenant au moins un actif sous forme d'huile et/ou au moins un actif contenu dans une huile-support non active ou une huile active, l'actif étant choisi parmi ceux ayant une action pharmaceutique se manifestant dans les couches supérieures de l'épiderme, pour l'obtention d'un médicament pour le traitement des couches supérieures de l'épiderme par application topique sur la peau, chaque application devant avoir une activité persistant pendant au moins une heure, les nanocapsules ayant des dimensions comprises entre 100 et 1 000 nm.

2. Utilisation selon la revendication 1, caractérisée par le fait qu'au moins un actif est choisi parmi ceux devant avoir sur la peau une action pharmaceutique de longue durée.

3. Utilisation selon l'une des revendications 1 ou 2, caractérisée par le fait que les nanocapsules ont des dimensions comprises entre 200 et 800 nm.

4. Utilisation selon l'une des revendications 1 à 3, caractérisée par le fait que le poids des nanocapsules chargées d'une phase huileuse utilisées constitue de 0,1 à 20 % en poids du médicament.

5. Utilisation selon l'une des revendications 1 à 4, caractérisée par le fait que le poids des nanocapsules représente de 0,5 à 10 % du poids total du médicament.

6. Utilisation selon l'une des revendications 1 à 5, caractérisée par le fait que les polymères non-biodégradables constitutifs des nanocapsules sont choisis dans le groupe formé par :
- les copolymères de chlorure de vinyle et d'acétate de vinyle ;
- les copolymères d'acide méthacrylique et d'ester méthylique d'acide méthacrylique ;
- l'acéto-phtalate de polyvinyle ;
- l'acéto-phtalate de cellulose ;
- le copolymère polyvinylpyrrolidone-acétate de vinyle réticulé ;
- les polyéthylènevinylacétates ;
- les oligomères styrène alkylalcool ;
- les copolymères d'acide et d'ester méthacrylique ;
- les polyacrylonitriles ;
- les polyacrylamides ;
- les polyéthylèneglycols ;
- les poly-(méthacrylate d'hydroxyéthyle) ;
- les dérivés de cellulose ;
- les polyamides ;
- les polyéthylènes ;
- le polypropylène ;
- les organopolysiloxanes ;
- la gélatine.

7. Utilisation selon l'une des revendications 1 à 6, caractérisée par le fait que la phase huileuse encapsulée dans les nanocapsules contient une huile active constituée par un filtre antisolaire UVA et/ou UVB et/ou une huile essentielle à activité bactéricide et/ou fongicide, une huile à activité humectante et une huile à activité antiradicaux libres.

8. Utilisation selon l'une des revendications 1 à 7, caractérisée par le fait que la phase huileuse encapsulée dans les nanocapsules contient une huilesupport choisie dans le groupe formé par les triglycérides simples ou modifiés et les huiles de silicone volatiles et les huiles minérales.

9. Utilisation selon l'une des revendications 1 à 8, caractérisée par le fait que la phase huileuse encapsulée dans les nanocapsules contient au moins un actif pris dans le groupe formé par les humectants, les antiacnéiques, les liporégulateurs, les antivieillissements, les colorants, les pigments, les émollients et les kératolytiques.

10. Utilisation selon l'une des revendications 1 à 9, caractérisée par le fait que les nanocapsules de polymère(s) non-biodégradable(s) sont mélangées à des nanocapsules de polymère(s) biodégradable(s).

11. Utilisation selon la revendication 10, caractérisée par le fait que les nanocapsules biodégradables contiennent au moins un actif à action immédiate sur la peau après application.

12. Utilisation selon l'une des revendications 10 à 11, caractérisée par le fait que les nanoparticules sont dans une composition sous forme de sérum, de lotion, de gel aqueux, hydroalcoolique ou huileux, d'émulsion eau dans l'huile ou huile dans l'eau ou de dispersions aqueuses de vésicules ioniques et/ou non-ioniques ou mixtes, pouvant ou non comporter une huile dispersée.

13. Procédé de traitement cosmétique des couches supérieures de l'épiderme par application topique sur la peau, caractérisé par le fait qu'on applique dans un véhicule approprié sur la peau des nanocapsules de polymères non-biodégradables encapsulant au moins un actif sous forme d'huile et/ou au moins un actif contenu dans une huile-support non active ou une huile active, l'actif étant choisi parmi ceux ayant une activité cosmétique sur les couches supérieures de l'épiderme, chaque application devant avoir une activité persistant au moins une heure sur les couches supérieures de l'épiderme, les nanocapsules ayant des dimensions comprises entre 100 et 1 000 nm.

14. Procédé selon la revendication 13, caractérisé par le fait qu'au moins un actif est choisi parmi ceux devant avoir sur la peau une action cosmétique de longue durée.

15. Procédé selon l'une des revendications 13 ou 14, caractérisé par le fait que les nanocapsules ont des dimensions comprises entre 200 et 800 nm.

16. Procédé selon l'une des revendications 13 à 15, caractérisé par le fait que le poids des nanocapsules chargées d'une phase huileuse utilisées constitue de 0,1 à 20 % en poids de la composition.

17. Procédé selon l'une des revendications 13 à 17, caractérisé par le fait que le poids des nanocapsules représente de 0,5 à 10 % du poids total de la composition.

18. Procédé selon l'une des revendications 13 à 17, caractérisé par le fait que les polymères non-biodégradables constitutifs des nanocapsules sont choisis dans le groupe formé par :
- les copolymères de chlorure de vinyle et d'acétate de vinyle ;
- les copolymères d'acide méthacrylique et d'ester méthylique d'acide méthacrylique ;
- l'acéto-phtalate de polyvinyle ;
- l'acéto-phtalate de cellulose ;
- le copolymère polyvinylpyrrolidone-acétate de vinyle réticulé ;
- les polyéthylènevinylacétates ;
- les oligomères styrène alkylalcool ;
- les copolymères d'acide et d'ester méthacrylique ;
- les polyacrylonitriles ;
- les polyacrylamides ;
- les polyéthylèneglycols ;
- les poly-(méthacrylate d'hydroxyéthyle) ;
- les dérivés de cellulose ;
- les polyamides ;
- les polyéthylènes ;
- le polypropylène ;
- les organopolysiloxanes ;
- la gélatine.

19. Procédé selon l'une des revendications 13 à 18, caractérisé par le fait que la phase huileuse encapsulée dans les nanocapsules contient une huile active constituée par un filtre antisolaire UVA et/ou UVB et/ou une huile essentielle à activité bactéricide et/ou fongicide, une huile à activité humectante et une huile à activité antiradicaux libres.

20. Procédé selon l'une des revendications 13 à 19, caractérisé par le fait que la phase huileuse encapsulée dans les nanocapsules contient une huilesupport choisie dans le groupe formé par les triglycérides simples ou modifiés et les huiles de silicone volatiles et les huiles minérales.

21. Procédé selon l'une des revendications 13 à 20, caractérisé par le fait que la phase huileuse encapsulée dans les nanocapsules contient au moins un actif pris dans le groupe formé par les humectants, les antiacnéiques, les liporégulateurs, les antivieillissements, les colorants, les pigments, les émollients et les kératolytiques.

22. Procédé selon l'une des revendications 13 à 21, caractérisé par le fait que la composition contient en outre des nanocapsules de polymères biodégradables.

23. Procédé selon l'une des revendications 13 à 22, caractérisé par le fait que les nanocapsules biodégradables contiennent au moins un actif à action immédiate sur la peau après application.

24. Procédé selon l'une des revendications 13 à 23, caractérisé par le fait que la composition est sous forme de sérum, de lotion, de gel aqueux, hydroalcoolique ou huileux, d'émulsion eau dans l'huile ou huile dans l'eau ou de dispersions aqueuses de vésicules ioniques et/ou non-ioniques ou mixtes, pouvant ou non comporter une huile dispersée.

## Claims

1. Use of nanocapsules of nonbiodegradable polymer encapsulating an oily phase containing at least one active ingredient in the form of an oil and/or at least one active ingredient in an inactive carrier oil or an active oil, the active ingredient being chosen from among those which have a pharmaceutical action exerted in the outer layers of the epidermis, to obtain a medicament for the treatment of the outer layers of the epidermis by topical application to the skin, each application needing to have an activity which persists for at least one hour, the nanocapsules being between 100 and 1000 nm in size.

2. Use according to Claim 1, characterised by the fact that at least one active ingredient is chosen from those which need to have a long-term pharmaceutical action on the skin.

3. Use according to either of Claims 1 and 2, characterised by the fact that the nanocapsules are between 200 and 800 nm in size.

4. Use according to one of Claims 1 to 3, characterised by the fact that the weight of the nanocapsules, charged with an oily phase, which are used constitutes 0.1 to 20% by weight of the medicinal product.

5. Use according to one of Claims 1 to 4, characterized by the fact that the weight of the nanocapsules represents 0.5 to 10% of the total weight of the medicinal product.

6. Use according to one of Claims 1 to 5, characterised by the fact that the constituent nonbiodegradable polymers of the nanocapsules are chosen from the group consisting of:
- vinyl chloride and vinyl acetate copolymers;
- methacrylic acid and methacrylic acid methyl ester copolymers;
- polyvinyl acetophthalate;
- cellulose acetophthalate;
- crosslinked polyvinylpyrrolidone-vinyl acetate copolymer;
- polyethylene vinyl acetates;
- styrene alkyl alcohol oligomers;
- methacrylic acid and ester copolymers
- polyacrylonitriles;
- polyacrylamides;
- polyethylene glycols;
- poly(hydroxyethyl methacrylate);
- cellulose derivatives;
- polyamides;
- polyethylenes;
- polypropylene;
- organopolysiloxanes;
- gelatin.

7. Use according to one of Claims 1 to 6, characterised by the fact that the oily phase encapsulated in the nanocapsules contains an active oil consisting of an agent for screening out solar UV-A and/or UV-B and/or an essential oil with bactericidal and/or fungicidal activity, an oil with humectant activity and an oil with anti-free radical activity.

8. Use according to one of Claims 1 to 7, characterised by the fact that the oily phase encapsulated in the nanocapsules contains a carrier oil chosen from the group consisting of simple or modified triglycerides and volatile silicone oils and mineral oils.

9. Use according to one of Claims 1 to 8, characterised by the fact that the oily phase encapsulated in the nanocapsules contains at least one active ingredient chosen from the group consisting of humectants, anti-acne agents, lipid regulators, anti-ageing agents, colorants, pigments, emollients and keratolytic agents.

10. Use according to one of Claims 1 to 9, characterised by the fact that the nanocapsules of nonbiodegradable polymer(s) are mixed with nanocapsules of biodegradable polymer(s).

11. Use according to Claim 10, characterised by the fact that the biodegradable nanocapsules contain at least one active ingredient with immediate action on the skin after application.

12. Use according to one of Claims 10 to 11, characterised by the fact that the nanoparticles are in a composition in the form of a serum, a lotion, an aqueous, dilute alcoholic or oily gel, a water-in-oil or oil-in-water emulsion or aqueous dispersions of ionic and/or nonionic or mixed vesicles which may or may not contain a dispersed oil.

13. Process for the cosmetic treatment of the outer layers of the epidermis via topical application to the skin, characterised by the fact that nanocapsules of nonbiodegradable polymers encapsulating at least one active ingredient in the form of an oil and/or at least one active ingredient in an inactive carrier oil or an active oil are applied to the skin in an appropriate vehicle, the active ingredient being chosen from among those which have a cosmetic activity on the outer layers of the epidermis, each application needing to have an activity which persists for at least one hour on the outer layers of the epidermis, the nanocapsules being between 100 and 1000 nm in size.

14. Process according to Claim 13, characterised by the fact that at least one active ingredient is chosen from those which need to have a long-term cosmetic action on the skin.

15. Process according to either of Claims 13 and 14, characterised by the fact that the nanocapsules are between 200 and 800 nm in size.

16. Process according to one of Claims 13 to 15, characterised by the fact that the weight of the nanocapsules, charged with an oily phase, which are used constitutes 0.1 to 20% by weight of the composition.

17. Process according to one of Claims 13 to 17, characterised by the fact that the weight of the nanocapsules represents 0.5 to 10% of the total weight of the composition.

18. Process according to one of Claims 13 to 17, characterised by the fact that the constituent nonbiodegradable polymers of the nanocapsules are chosen from the group consisting of:
- vinyl chloride and vinyl acetate copolymers;
- methacrylic acid and methacrylic acid methyl ester copolymers;
- polyvinyl acetophthalate;
- cellulose acetophthalate;
- crosslinked polyvinylpyrrolidone-vinyl acetate copolymer;
- polyethylene vinyl acetates;
- styrene alkyl alcohol oligomers;
- methacrylic acid and ester copolymers
- polyacrylonitriles;
- polyacrylamides;
- polyethylene glycols;
- poly(hydroxyethyl methacrylate);
- cellulose derivatives;
- polyamides:
- polyethylenes;
- polypropylene;
- organopolysiloxanes;
- gelatin.

19. Process according to one of Claims 13 to 18, characterised by the fact that the oily phase encapsulated in the nanocapsules contains an active oil consisting of an agent for screening out solar UV-A and/or UV-B and/or an essential oil with bactericidal and/or fungicidal activity, an oil with humectant activity and an oil with anti-free radical activity.

20. Process according to one of Claims 13 to 19, characterised by the fact that the oily phase encapsulated in the nanocapsules contains a carrier oil chosen from the group consisting of simple or modified triglycerides and volatile silicone oils and mineral oils.

21. Composition according to one of Claims 13 to 20, characterised by the fact that the oily phase encapsulated in the nanocapsules contains at least one active ingredient chosen from the group consisting of humectants, anti-acne agents, lipid regulators, anti-ageing agents, colorants, pigments, emollients and keratolytic agents.

22. Process according to one of Claims 13 to 21, characterized by the fact that the composition furthermore contains nanocapsules of biodegradable polymers.

23. Process according to one of Claims 13 to 22, characterised by the fact that the biodegradable nanocapsules contain at least one active ingredient with immediate action on the skin after application.

24. Process according to one of Claims 13 to 23, characterised by the fact that the composition is provided in the form of a serum, a lotion, an aqueous, dilute alcoholic or oily gel, a water-in-oil or oil-in-water emulsion or aqueous dispersions of ionic and/or nonionic or mixed vesicles which may or may not contain a dispersed oil.

## Patentansprüche

1. Verwendung von Nanokapseln aus biologisch nicht abbaubarem Polymer, in die eine Ölphase eingekapselt ist, die mindestens einen Wirkstoff in Form von Öl und/oder mindestens einen in einem nicht aktiven Trägeröl oder in einem aktiven Öl enthaltenen Wirkstoff enthält, der aus denjenigen ausgewählt ist, die eine sich in den oberen Schichten der Epidermis manifestierende pharmazeutische Wirkung haben, zur Herstellung eines Arzneimittels für die Behandlung der oberen Schichten der Epidermis durch topische Anwendung auf der Haut, wobei jede Anwendung eine mindestens eine Stunde anhaltende Wirkung haben muß und die Nanokapseln Abmessungen von 100 bis 1.000 nm haben.

2. Verwendung nach Anspruch 1, dadurch gekennzeichnet, daß mindestens ein Wirkstoff aus denjenigen ausgewählt ist, die eine lang anhaltende pharmazeutische Wirkung auf die Haut haben müssen.

3. Verwendung nach einem der Ansprüche 1 oder 2, dadurch gekennzeichnet, daß die Nanokapseln Abmessungen von 200 bis 800 nm haben.

4. Verwendung nach einem der Ansprüche 1 bis 3, dadurch gekennzeichnet, daß das Gewicht der verwendeten, mit einer Ölphase gefüllten Nanokapseln 0,1 bis 20 Gew.-% des Arzneimittels darstellt.

5. Verwendung nach einem der Ansprüche 1 bis 4, dadurch gekennzeichnet, daß das Gewicht der Nanokapseln 0,5 bis 10 % des Gesamtgewichts des Arzneimittels darstellt.

6. Verwendung nach einem der Ansprüche 1 bis 5, dadurch gekennzeichnet, daß die die Nanokapseln bildenden biologisch nicht abbaubaren Polymere aus der Gruppe ausgewählt sind, die gebildet ist von:
- Vinylchlorid- und Vinylacetat-Copolymere;
- Methacrylsäure- und Methacrylsäuremethylester-Copolymere;
- Polyvinylacetophtalat;
- Celluloseacetophtalat;
- Polyvinylpyrrolidon-vernetztes Vinylacetat-Copolymere;
- Polyethylenvinylacetate;
- Styrolalkylalkohol-Oligomere;
- Methacrylsäure-und Methacrylester-Copolymere;
- Polyacrylonitrile;
- Polyacrylamide;
- Polyethylenglycole;
- Poly-(hydroxyethylmethacrylat);
- Cellulosederivate;
- Polyamide;
- Polyethylene;
- Polypropylen;
- Organopolysiloxane;
- Gelantine.

7. Verwendung nach einem der Ansprüche 1 bis 6, dadurch gekennzeichnet, daß die in den Nanokapseln eingekapselte Ölphase ein aus einem UVA- und/oder UVB-Sonnenschutzfilter bestehendes aktives Öl und/oder ein etherisches Öl mit bakterizider und/oder fungizider Wirkung, ein Öl mit anfeuchtender Wirkung und ein Öl mit einer Wirkung gegen freie Radikale enthält.

8. Verwendung nach einem der Ansprüche 1 bis 7, dadurch gekennzeichnet, daß die in den Nanokapseln eingekapselte Ölphase ein Trägeröl enthält, das aus der Gruppe ausgewählt ist, die aus den einfachen oder modifizierten Triglyceriden und den flüchtigen Silikonölen und den Mineralölen besteht.

9. Verwendung nach einem der Ansprüche 1 bis 8, dadurch gekennzeichnet, daß die in den Nanokapseln eingekapselte Ölphase mindestens einen Wirkstoff enthält, der aus der Gruppe genommen ist, die aus den Anfeuchtungsmitteln, den Aknemitteln, den Fettreglern, den Mitteln gegen Altern, den Farbstoffen, den Pigmenten, den Erweichungsmitteln und den Keratolyten gebildet ist.

10. Verwendung nach einem der Ansprüche 1 bis 9, dadurch gekennzeichnet, daß die Nanokapseln aus biologisch nicht abbaubarem Polymer bzw. biologisch nicht abbaubaren Polymeren mit Nanokapseln aus biologisch abbaubarem Polymer bzw. biologisch abbaubaren Polymeren gemischt sind.

11. Verwendung nach Anspruch 10, dadurch gekennzeichnet, daß die biologisch abbaubaren Nanokapseln mindestens einen Wirkstoff mit unmittelbarer Wirkung auf die Haut nach Anwendung enthalten.

12. Verwendung nach einem der Ansprüche 10 bis 11, dadurch gekennzeichnet, daß die Nanopartikel in einer Zusammensetzung in Form von Serum, Lotion, wässrigem, hydroalkoholischem oder öligem Gel, Wasser-in-Öl- oder Öl-in-Wasser-Emulsion oder wässrigen Dispersionen von ionischen und/oder nichtionischen oder gemischten Bläschen sind, die ein dispergiertes Öl enthalten können oder nicht.

13. Verfahren zur kosmetischen Behandlung der oberen Schicht der Epidermis durch topische Anwendung auf der Haut, dadurch gekennzeichnet, daß man auf die Haut in einem geeigneten Träger Nanokapseln aus biologisch nicht abbaubaren Polymeren aufträgt, in die mindestens ein Wirkstoff in Form von Öl und/oder mindestens ein in einem nicht aktiven Trägeröl oder in einem aktiven Öl enthaltener Wirkstoff eingekapselt ist, der aus denjenigen ausgewählt ist, die eine kosmetische Wirkung auf die oberen Schichten der Epidermis haben, wobei jede Anwendung eine mindestens eine Stunde andauernde Wirkung auf die oberen Schichten der Epidermis haben muß und die Nanokapseln Abmessungen von 100 bis 1.000 nm haben.

14. Verfahren nach Anspruch 13, dadurch gekennzeichnet, daß mindestens ein Wirkstoff aus denjenigen ausgewählt ist, die eine lang anhaltende kosmetische Wirkung auf die Haut haben müssen.

15. Verfahren nach einem der Ansprüche 13 oder 14, dadurch gekennzeichnet, daß die Nanokapseln Abmessungen von 200 bis 800 nm haben.

16. Verfahren nach einem der Ansprüche 13 bis 15, dadurch gekennzeichnet, daß das Gewicht der verwendeten, mit einer Ölphase gefüllten Nanokapseln 0,1 bis 20 Gew.-% der Zusammensetzung darstellt.

17. Verfahren nach einem der Ansprüche 13 bis 17, dadurch gekennzeichnet, daß das Gewicht der Nanokapseln 0,5 bis 10 % des Gesamtgewichts der Zusammensetzung darstellt.

18. Verfahren nach einem der Ansprüche 13 bis 17, dadurch gekennzeichnet, daß die die Nanokapseln bildenden biologisch nicht abbaubaren Polymere ausgewählt sind aus der Gruppe, die gebildet ist von:
- Vinylchlorid- und Vinylacetat-Copolymere;
- Methacrylsäure-und Methacrylsäuremethylester-Copolymere;
- Polyvinylacetophtalat;
- Celluloseacetophtalat;
- Polyvinylpyrrolidon-vernetztes Vinylacetat-Copolymere;
- Polyethylenvinylacetate;
- Styrolalkylalkohol-Oligomere;
- Methacrylsäure-und Methacrylester-Copolymere;
- Polyacrylonitrile;
- Polyacrylamide;
- Polyethylenglycole;
- Poly-(hydroxyethylmethacrylat);
- Cellulosederivate;
- Polyamide;
- Polyethylene;
- Polypropylen;
- Organopolysiloxane;
- Gelantine.

19. Verfahren nach einem der Ansprüche 13 bis 18, dadurch gekennzeichnet, daß die in den Nanokapseln eingekapselte Ölphase ein aus einem UVA- und/oder UVB-Sonnenschutzfilter bestehendes aktives Öl und/oder ein etherisches Öl mit bakterizider und/oder fungizider Wirkung, ein Öl mit anfeuchtender Wirkung und ein Öl mit Wirkung gegen frei Radikale enthält.

20. Verfahren nach einem der Ansprüche 13 bis 19, dadurch gekennzeichnet, daß die in den Nanokapseln eingekapselte Ölphase ein Trägeröl enthält, das aus der Gruppe ausgewählt ist, die von den einfachen oder modifizierten Triglyceriden und den flüchtigen Silikonölen und den Mineralölen gebildet ist.

21. Verfahren nach einem der Ansprüche 13 bis 20, dadurch gekennzeichnet, daß die in den Nanokapseln eingekapselte Ölphase mindestens einen Wirkstoff enthält, der aus der Gruppe genommen ist, die aus den Anfeuchtungsmitteln, den Aknemitteln, den Fettreglern, den Mitteln gegen Altern, den Farbstoffen, den Pigmenten, den Erweichungsmitteln und den Keratolyten gebildet ist.

22. Verfahren nach einem der Ansprüche 13 bis 21, dadurch gekennzeichnet, daß die Zusammensetzung außerdem Nanokapseln aus biologisch abbaubaren Polymeren enthält.

23. Verfahren nach einem der Ansprüche 13 bis 22, dadurch gekennzeichnet, daß die biologisch abbaubaren Nanokapseln mindestens einen Wirkstoff mit unmittelbarer Wirkung auf die Haut nach Anwendung enthalten.

24. Verfahren nach einem der Ansprüche 13 bis 23, dadurch gekennzeichnet, daß die Zusammensetzung in Form von Serum, Lotion, wässrigem, hydroalkoholischem oder öligem Gel, Wasser-in-Öl- oder Öl-in-Wasser-Emulsion oder Dispersionen von ionischen und/oder nichtionischen oder gemischten wässrigen Bläschen ist, die ein dispergiertes Öl enthalten können oder nicht.
